# EUROPEAN PATENT APPLICATION

(11) **EP 4 555 964 A1**
(43) Date of publication of application: **21.05.2025**
(21) Application number: 23210093.3
(22) Date of filing: 15.11.2023
(51) Int. Cl.: A61B 90/00, A61B 5/00, A61B 5/026, A61B 17/00, A61B 34/20

(54) **PROJECTION APPARATUS FOR ANALYSING OBJECTS OF INTEREST ON OR OVER A SURFACE OF A SUBJECT**

(71) Applicant: Stichting Radboud universitair medisch centrum, 6525 GA Nijmegen (NL)
(72) Inventor: Hummelink, Stefan Laurentius Maria, Nijmegen (NL)
(74) Representative: Nederlandsch Octrooibureau

(57) **Abstract**

An apparatus comprising a detector having a detecting field and configured to detect at least part of a subject in the detecting field and detect an object in the detecting field; and a processor configured to identify a command based on the detected object, wherein the command designates a region on the surface of the subject; determine an attribute of a tissue of the subject within the designated region; and output an indication of the determined attribute.

## Description

### Field of the invention

The present invention relates to an apparatus comprising a detector having a detecting field located in front of a subject and configured to detect an object in the detecting field; and a processor configured to identify a command from the detected object, determine an attribute of a tissue of the subject and output an indication of the determined attribute.

### Background

For surgical procedures, it is important to localize features, which may be visible on e.g., medical images, in the patient's body.

US 9510914 B2 describes surgical guidance and image registration are provided, in which three-dimensional image data associated with an object or patient is registered to topological image data obtained using a surface topology imaging device.

US 9646423 B1 describes a method for providing augmented reality in minimally invasive surgery includes capturing pre-operative image data of internal organs of a patient, capturing intra-operative image data of the internal organs with an endoscope during a surgical procedure, registering the pre-operative image data and the intra-operative data in real time during the surgical procedure, tracking the position and orientation of the endoscope during the surgical procedure, and augmenting the intra-operative image data captured by the endoscope in real time with a rendering of at least a portion of an internal organ of the patient that is in registration with the real time intra-operative image data from the endoscope but outside of the field of view of the endoscope.

WO 2015/135985 A1 describes a projection system for image projection of features on a subject. Intraoperative position markers are disposed on a first body surface, and a moveable first detector with a detection line of sight is provided.

The prior art document has the disadvantage of the need for position markers on the subject. Additionally, the prior art may involve calibrating a projected image with a detected image, which may be cumbersome and/or reduce accuracy and/or quality of the projected image. Moreover, there is a need for an improved imaging tool that is able to provide relevant information about a subject.

### Summary of the invention

The present invention seeks to provide an apparatus for detecting one or more objects of interest on or over a surface of a subject, designate a region on the surface of the subject and determine one or more attributes of tissues within the designated region.

According to an aspect of the present invention, an apparatus comprises a detector having a detecting field located in front of a subject and configured to detect at least part of a subject in the detecting field and detect an object in the detecting field; and a processor configured to: identify a command based on the detected object, wherein the command designates a region on the surface of the subject; determine an attribute of a tissue of the subject within the designated region; and output an indication of the determined attribute.

The apparatus disclosed herein may be designed to detect an object in a field of view of a detector. The objects detected may be outside, in front, or on a surface of a subject. Depending on the detected object, the processor may identify an internal command that designates a region on the surface of the subject, the designated region may also include tissues below the surface of the subject, such as internal tissues and/or organs. After a region has been designated on the surface of the subject, the processor is configured to determine an attribute of a tissue of the subject within the designated region. In this context, determining the attribute of the tissue refers to determining the attribute of superficial and/or deep tissues of the subject. Thus, the attribute is any measurable quality or characteristic that the tissue in the designated region may have.

The processor outputs an indication of the determined attribute, for example on a display or by projection on the subject's surface, or by means of an auditive signal. In this manner the determination and output of the attribute, may refine the practitioner's understanding of the subject tissue and/or body composition or properties during the procedure, particularly for attributes that are challenging to visually evaluate or entirely imperceptible. In general, the apparatus's ability to determine and output attributes of subject tissues within designated regions improves the precision of medical procedures by providing information to assist medical practitioners to make well-informed decisions. The apparatus is configured to detect objects external to the subject (e.g., hand of surgeon or instrument) or objects pertaining to the subject, such as anatomical features (e.g., organs) and/or physiological features, as further explained below.

The apparatus of any preceding claim, further comprising a projector configured to project on the surface of the subject an indication of the designated region and/or the indication of the determined attribute of the tissue. The projector acts as a visual aid that enables an easy identification of critical regions of the subject by projecting indications of designated regions and tissue attributes directly onto the subject's surface. This visual overlay contributes to improved instrument handling, ensuring precise placement and maneuvering. The projector reduces the need for medical practitioners to divert their attention to external screens, allowing them to visualize attributes without needing to shift focus away from the immediate procedural environment.

The object may be external to the subject, and wherein the object may indicate a point of a shape of the designated region, and the processor may be configured to: detect successive points, on the surface of the subject, indicated by the object as the object moves with respect to the subject; and connect the successive points to determine the shape of the designated region. In this manner the tissue of the subject within the designated region is precisely defined by an object indication provided by the detected external object. This improves the efficiency of the apparatus to provide on-demand information to a user. Furthermore, connecting the successive points enables real-time reconstruction of shapes of the designated region, which facilitates a precise determination of the attribute of the tissue of the subject within the designated region.

The projector may be configured to project an indication of the shape of the designated region insofar the points on the surface of the subject have been detected. This real-time representation of the designated region enhances user understanding and communication with the apparatus. Users, such as medical practitioners, can interactively draw or manipulate the shape of the designated region, ensuring precision and guiding them throughout the procedure. This feature not only improves user confidence but also facilitates effective communication and collaboration between the user and the apparatus, ultimately enhancing the quality and safety of the procedure.

The shape of the designated region may be a closed shape or a nearly closed shape, wherein determining the attribute of the tissue may comprise determining the attribute of superficial and/or deep tissue of the subject on and/or below an area defined by the designated region. The shape of the designated region may be an open shape, a closed shape, or a nearly closed shape and they may be used for different purposes. By defining a shape that is closed or nearly closed, the processor determines tissue attributes specific to an enclosed area, which the processor will identify with attributes such as area, volume, etc. An open shape may be used to cause the processor to identify another command for making alternative calculations, such as linear calculation (e.g., length, curvature, angle, etc. of an open shape) or for providing a request for a recommendation during a medical procedure, for example, an open shape may request the apparatus to give a recommendation to close or treat a wound.

The detector may be configured to detect at least part of a hand or an instrument as the object. This enables intuitive interaction through hand gestures and postures, simplifying the user communication with the apparatus and making it more user-friendly. Moreover, as the user can draw without touching the surface of the subject, this promotes hands-free control, which reduces the risk of cross-contamination at the operation field.

In other situations, in which the object is a part of a body of the subject, the processor is further configured to determine a shape of the object within the designated region. This enhances user interaction by eliminating the need for manual shape input or complex procedures, making the system more user-friendly and efficient. Furthermore, the ability of the processor to determine the shape of an object when that object is a part of the subject's body, offers the advantage enabling convenient and automatic identification of the shape of the object without the user's input, specifically convenient when multiple objects are identified. Additionally, the automated determination of the shape can provide highly precise measurements and annotations of body tissues and structures.

The attribute of the tissue may comprise one or more of: name, temperature , size, area, volume, location, and type of tissue. By comprising a wide range of attributes the apparatus achieves a comprehensive understanding of tissue characteristics. Attributes like temperature, size, area, and volume assist medical practitioners with specific and quantifiable data. This precision ensures interventions are tailored to each patient's unique anatomical attributes. Attributes such as location of body parts contribute to spatial awareness, as practitioners can precisely pinpoint tissue areas for targeted actions, minimizing invasiveness and potential collateral damage. Attributes such as tissue type and name enhances differentiation and identification of specific tissue structures, which facilitates distinguishing between various tissue types to avoid errors and complications during procedures. Attributes like location and size, when presented in real-time, guide medical practitioners during interventions. This advantage directly aligns with the dynamic nature of medical procedures, providing timely and relevant insights for immediate actions.

The processor may be configured to determine the attribute of visceral, epithelial, connective, adipose, muscle, nervous, or abnormal tissue of the subject. Differentiation of tissue types also enhances precision during the procedure to prevent damage of tissue that is not targeted. For example, when removing a tumor on an internal organ. Furthermore, different tissue types often require distinct treatment strategies. Determining tissue types ensures that treatments and medical procedures are tailored to the characteristics of the tissues being addressed.

The attribute of the tissue may be determined based on real-time measurements of the designated region and/or based on mapping the designated region on preoperative images containing data representing the subject.

Determining tissue attributes based on real-time measurements of the designated region offers immediate insights during medical procedures and enables medical practitioners to receive immediate feedback. Real-time measurements are particularly valuable for assessing dynamic aspects of tissues, such as blood flow, temperature, or electrical conductivity, which can change rapidly during procedures. The real-time measurements may be performed by the apparatus, e.g. by the detector. Alternatively, real-time measurements may be performed by an external device in communication with the processor.

Determining tissue attributes based on preoperative image allow for more detailed information or attributes of anatomical structures that cannot be captured by the real-time measurements, which usually comprise more structural and/or functional information of superficial and deep tissues. The combination of real-time measurements and preoperative images can enhance the accuracy and effectiveness of medical interventions by providing both immediate feedback and detailed anatomical insights. In some examples, real-time measurements comprise an intraoperative imaging device acquiring data representing the subject. The imaging device may involve one or more medical imagining techniques. For example, fluoroscopy, computed tomography, magnetic resonance imaging, ultrasound imaging, positron emission tomography, single-photon emission computed tomography.

The preoperative image dataset may comprise images from one or more medical imaging techniques, for example those mentioned above. Different imaging techniques contribute to characterizing different types of tissues. CT scans may be used for visualizing bones and dense structures, while MRI can be used for capturing soft tissue details. Ultrasound provides real-time imaging with no radiation exposure, making it suitable for various applications. PET and SPECT are essential for functional imaging and detecting metabolic activity. By incorporating these diverse imaging modalities, medical practitioners can gain a comprehensive, three-dimensional understanding of the subject. Furthermore, combining imaging datasets can help on detecting abnormalities that might be missed when relying on a single imaging source, ultimately leading to more precise diagnoses and treatment plans. Finally, if multiple imaging techniques are used the apparatus can assist in a wide range of medical procedures. For example, surgery, radiation therapy planning, or interventional procedures.

The attribute of the tissue may be determined by a recognition algorithm comprising Artificial Intelligence (Al).

AI algorithms may provide data-driven insights that may enhance the accuracy and efficiency on determining the attribute of the tissue. Furthermore, their adaptability can ensure that the apparatus continually improves its attribute determination accuracy over time. Leading to a more robust apparatus adept at recognizing and characterizing different tissue types, including anomalies or abnormalities

The attribute of the tissue may be determined momentarily and/or over time. The option to determine tissue attributes before the procedure begins supports pre-procedural planning. This advantage enables medical practitioners to strategize interventions. Meanwhile, the ability to determine attributes over time provides dynamic insights during the procedure, offering real-time updates on tissue attributes as the procedure advances, assisting in decision-making and intervention adjustments.

The detector may comprise an image sensor and/or a position sensor to detect a position of the object. An image sensor in the detector facilitates to later recreate visual indications and allows the apparatus to capture real-time visual information, facilitating the output to medical practitioners with direct visual feedback during procedures. A position sensor can provide a more accurate position and orientation of the object in the detecting field which is advantageous during medical procedures that require high accuracy. The combination of an image sensor and a position sensor facilitates the integration of visual and spatial information enhancing precision. Combining an image sensor with a position sensor allows the apparatus to integrate visual and spatial data, which may be valuable for guiding surgical instruments or ensuring that treatments are delivered to the exact target.

The apparatus may further comprise a memory to store the determined attribute of the tissue for future reference and/or analysis. The memory facilitates the storage of attribute-related information for subsequent reference and/or in-depth analysis. It may also hold instructions for the processor to follow.

The detector may be able to detect multiple objects simultaneously, and the processor may be configured to identify commands and determine attributes for each detected object individually. Detecting multiple objects, such as instruments or different tissues and organs, facilitates the medical procedures when more than one instrument is present in the detecting field or when more than one attribute is to be determined during the medical procedure. For example, during surgery, the apparatus can monitor both the target organ and surrounding tissues simultaneously.

The processor may be further configured to analyze the attribute of the tissue and provide a recommendation for a procedure based on the analysis. By offering recommendations based on attribute analysis, the apparatus contributes to improved procedural outcomes. If analyzing tissue attributes and providing procedure recommendations is data-driven, the recommendations can be based on objective data, enhancing the precision and effectiveness during a medical procedure.

The processor is configured to generate a graphical representation of the determined attribute and cause the projector to project the graphical representation onto the surface of the subject. Graphical representations of determined attributes allows users to understand complex attribute information in a visual and easily understandable way. Furthermore, projected graphical representations can enable users to see the immediate impact of their interventions.

### Brief Description of the Drawings

The present invention will be explained in further detail hereinafter based on a number of exemplary embodiments with reference to the drawings. The drawings are diagrammatic and may not be drawn to scale. Throughout the drawings, similar items may be marked with the same reference numerals. In the drawings:
FIG. 1A schematically shows an apparatus according to the present disclosure;
FIG. 1B schematically shows the apparatus of FIG. 1A, wherein the detected object pertains to the subject.
FIG. 1C schematically shows the apparatus of FIG. 1A, wherein the detected object is external to the subject.
FIG. 2A schematically shows another apparatus according to the present disclosure and comprising a projector.
FIG. 2B schematically shows the apparatus of FIG. 2A. projecting an indication of the attribute of the tissue of the subject.
FIG. 2C schematically shows the apparatus of FIG. 2A assisting during a medical procedure.

### Detailed Description

In medicine, various difficulties arise regarding identifying patient information, particularly during semi-invasive and invasive medical procedures such as surgeries and biopsies, etc. For example, identifying and localizing patient vital signs such as temperature, blood pressure, heart rate, etc.; blood flow; burns; infections; rheumatism; cancerous cells; microorganisms; and more. For example, before or during surgery, it may be advantageous to identify objects, for example, parts of a patient's vascular system and/or internal tissues or organs and determine attributes of these objects to provide more information about the patient's tissues to the medical practitioner. Furthermore, it may be advantageous to also detect or identify other objects external to the patient to assist the medical practitioner before or during surgery.

The apparatus disclosed herein is designed to detect an object in the field of view of a detector. The objects detected may be outside, in front of, or on a surface of a subject. The apparatus may be located at a reasonable working distance from the subject, for example, up to 3 meters, to not interfere with medical procedures performed on the subject.

It is noted that the term 'detector field of view' may denote herein an area or volume that the detector is capable of detecting. The field of view may have a conical shape with a center (the apex) at the detector. It is further noted that the detector may be capable of detecting radiation and/or signals that are emitted or reflected by objects external to the subject or by objects on the surface or below the surface of the subject. For example, detecting a skin burn, at least part of an internal organ or a vascular structure below the surface of the subject.

It is further noted that the surface of the subject (e.g., the skin of a patient) may not typically be a flat surface and may comprise a three-dimensional profile (e.g., it may be curved and may have upstanding walls, recesses, etc.) following body and organ curvatures and the like. The detector may detect objects within the field of view in a two-dimensional (2D) fashion.

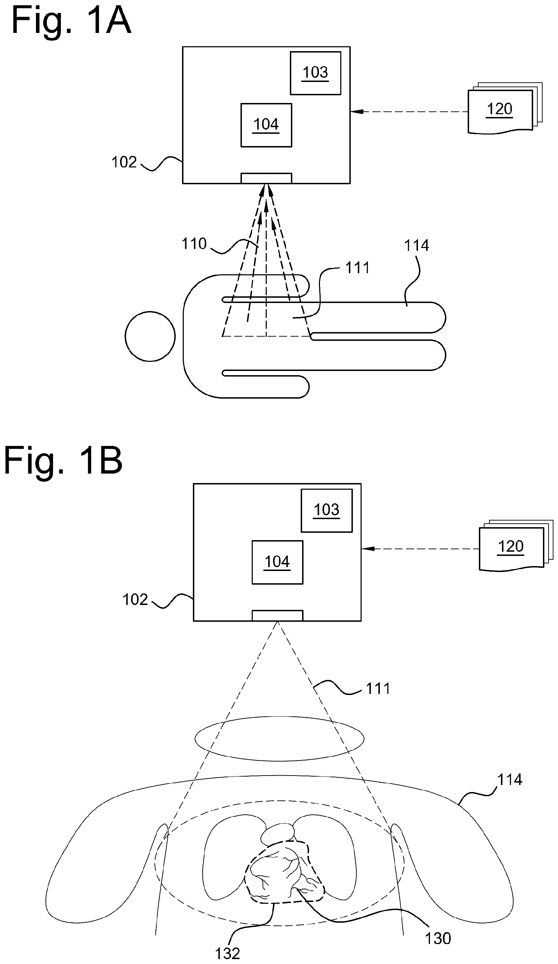

FIG. 1A schematically shows an apparatus 102 according to the present disclosure. Subject 114 is not part of the image projection apparatus 102. The apparatus 102 includes a detector 104 configured to detect objects external to the subject (e.g., the hand of the surgeon or an instrument) or objects pertaining to the subject, such as anatomical features (e.g., organs) and/or physiological features. Detector 104 may generate a detected signal and/or image based on detected radiation beams 110 from the detector field of view 111. The detected radiation beams 110 may be emitted or reflected by the detected objects. Apparatus 102 may include more than one detector 104, and each detector may generate detected images and/or signals. The detector 104 may be a position or an image detector and may comprise a sensor. For example, the image sensor may be a complementary metal-oxide-semiconductor (CMOS) detector detecting visible light beams. It will be understood that other sensors may be appropriate depending on the wavelength range of the detected radiation beams 110. Additionally, or optionally, appropriate filters may be provided so that the detector 104 is configured to sense radiation in a specific bandwidth.

The detector 104 may include a light emitter configured to emit radiation onto the field of view of the detector and to detect light reflecting on the object and/or the surface of the subject when exposed to the emitted light. The emitted light may be configured for a certain frequency. This may be helpful for imaging techniques, including, but not limited to, fluorescent dyes, laser speckle contrast imaging (LSCI), and laser Doppler imaging. For example, certain bacteria or tissue may emit light at a certain frequency when exposed to light from the light emitter. In another example, the apparatus may be configured to perform LSCI with a laser that emits light. Additionally, the emitted light may excite certain bacteria or tissues of interest when exposed to the emitted light.

In the particular example that the detector 104 is a Laser Doppler Imaging (LDI) system. The LDI system may include a laser exciter, a laser, and a camera configured to detect a change over time. The LDI system may be configured to detect a blood vessel as an object and subsequently detect the movement of red blood cells in the blood vessel by reflecting a specific wavelength to determine the movement of red blood cells in the blood vessel. In another example, when using laser speckle contrast imaging (LSCI), a laser light reaches the object, in this case, the blood vessel, and the moving blood cells generate Doppler components in the reflected light. LSCI may also be configured to identify a burn as an object and determine a burn depth in a subject. The red blood cells may scatter the coherent light to cause a speckle pattern to blur. Red blood cell movement may be determined based on the degree of the blur in the detected image. The burn depth can be determined based on the presence of red blood cell movement.

Typically, the surface of the subject 114 is a skin surface. However, the surface of the subject 114 may alternatively be an internal body surface of a subject in certain applications of apparatus 102. For example, after surgery has begun and subject 114 has an open cavity, the object may be detected from inside of the subject within the open cavity.

The apparatus 102 may include a processor 103 configured to identify a command from the detected object. Depending on the detected object, the command designates a region on the surface of the subject. When the object is a body part pertaining to the subject, the processor identifies a command from the detected body part to designate a region associated with the detected body part.

FIG. 1B schematically shows the apparatus of FIG. 1A and wherein the detected object 130 pertains to the subject 114. In the example of Fig. 1B the processor 103 will identity a command to designate a region on the surface of the subject 114, where a blood vessel on or in the heart is the detected object 130. In the depicted example, the designated region 132 is the heart of the subject 114. In another example, if the detected object is a burn, the processor will identify the command to designate a burned region of the skin as designated region. In a further example, if the object is an internal organ that is at least partially exposed from the subject's cavity, the processor may designate, as designated region, a surface of the internal organ in front of the detector. In another example, if the object is a subcutaneous section of the skin (e.g., an open skin flap), the identified command will designate a subcutaneous surface of the skin containing epithelial, adipose, vascular tissue, etc. In a further example, the detected object is a marker on the surface of the subject. The marker may be a physical or an optical marker, such as a fluorescent marker to identify abnormal cells (e.g., cancerous cells) and the designated region may be an organ of the subject having a tumor.

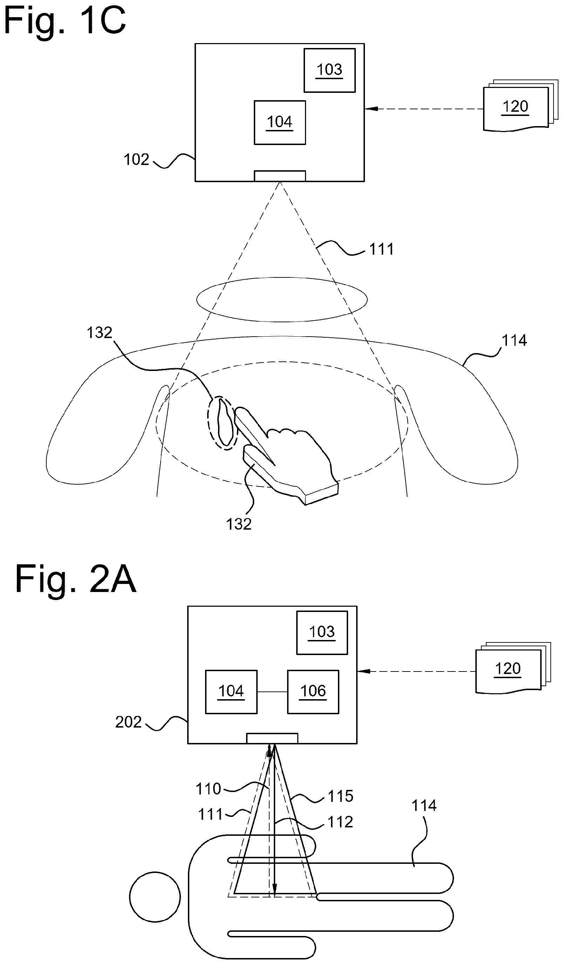

In the case that the detected object is external to the subject 114, for instance an instrument, the processor 103 will designate a region on the surface of the object indicated by the external object. Details on the detected objects are described with reference to FIG. 1C.

After a region has been designated on the surface of the subject, the processor 103 is configured to determine an attribute of a tissue of the subject within the designated region. For instance, the tissue may be superficial tissue, blood vessel tissue, internal organ tissue or any other tissue within the designated region. Thus, the attribute is any measurable quality or characteristic that the tissue in the designated may have. For example, the attribute maybe a superficial shape, temperature, surface area (which may be measured in, e.g., square centimeters, cm²) or volume (which may be measured in, e.g., cubic centimeters, cm³) of a burn, an internal organ, or a subcutaneous skin section. The attribute may also be the location of the object with respect to another identified object or with respect to another part of the subject. For instance, as shown in Fig. 1B, the attribute may be the location, length, or flow of blood vessels of the heart. The attribute may also be the type of tissue and/or composition of the tissue within the designated region. For instance, the attribute may be the one or more tissues such as visceral, epithelial, connective, adipose, muscle, nervous, or abnormal tissue. In this context, abnormal tissue may be inflamed, infected, or damaged tissue, or tissue that has abnormally grown (e.g., tumor). The attribute to be determined may be chosen by a user by a user interface or may be linked to the detected object, the command or the characteristics of the detector. The attribute may also be automatically chosen by the apparatus depending on the ongoing medical procedure, in which the medical procedure may also be determined by the processor based on the signals detected by the detector. The skilled person will understand that the determined attribute is directly associated with the type of detector used. In addition to LDI and LSCI mentioned before, the detector 104 may detect and measure (near-)infrared, ultrasound, fluoroscopic, photoacoustic, electrical impedance, spectroscopic, magnetic signals/images in real time. Alternatively, or additionally, the detector 104 may be an image detector, e.g., visible-light camera, and the processor 103 may be configured to use image processing, and/or computer vision algorithms to determine the attribute of the tissue. For example, the processor 103 may use Artificial intelligence-based recognition and/or computer vision algorithms to continuously or intermittently scan the detecting field of view to detect the objects and identify the command from the detected object.

Alternatively, or additionally, the attribute of the tissue is determined by the processor 103 based on a mapping algorithm to map the designated region 130 on preoperative images 120 containing data representing the subject 114. These images may be acquired by any medical imaging technique known in the art, for example, computed tomography, magnetic resonance imaging, ultrasound imaging, positron emission tomography, single-photon emission computed tomography, etc. Preoperative images 120 may simplify, complement or enhance the determination of the attribute, that was based on the measurements of the detector 104, in order to provide a more precise or detailed information of the tissues within the designated region 130. For example, when the detected object is a burn, the attribute of the volume of burned tissue maybe complemented with preoperative images to identify further damage below the skin. Similarly, preoperative images 120 may provide a more precise location of the detected blood vessels of the heart with respect to the surface of the subject. In the example of the detected subcutaneous section of the skin, preoperative images 120 may provide information of the subcutaneous volume of fat (cm³). In another example, preoperative images 120 such as PET scans may complement spectroscopic measurements to identify location and composition of tumors, particularly when they are not visually accessible for the medical practitioner during surgery.

In application in which the detector is an image detector, the images may only detect shapes and colors that the processor 103 will use to determine attributes, for example, to identify and classify detected objects based on the preoperative images 120. For instance, the image detector may detect several organs or tissues in an open cavity (designated region) and generate an image. Subsequently, the processor 103 may map the image on MRI scans to identify one or more tissues or organs in the image. In this example, the attribute may be the name of the anatomical structure or organ within the designated region 132 (e.g., aorta, left lung, etc.).

As mentioned before the objects detected by detector 104 may be outside, in front, or on a surface of a subject. Depending on the detector the object may be in or below the surface of the subject. In general objects on/in/below the surface of the subject are objects pertaining to the subject, such as body parts, organs, skin sections, etc. As described before. Objects outside or in front of the surface of the subject may generally be external to the subject and may be detected to allow interaction of the apparatus 102 with e.g., a medical practitioner or a robot performing a medical procedure. Examples of external objects may include surgical and medical instruments held by a human (medical practitioner) such as pens, probes, forceps, scalpels, and other objects for example a human hand or an end-effector of a robot, such as a gripper, forceps, etc.

FIG. 1C schematically shows the apparatus of FIG. 1A and wherein the detected object is external to the subject. As described above, when the object is a body part pertaining to the subject, the processor identifies a command from the detected body part to designate a region associated to the detected object. In the situation that the object is external to the subject 114, the processor 103, based on the external object, will identify a command to designate a region on the surface of the subject. As shown in Fig. 1C, if the object 130 is a hand over the surface of the subject 114, the processor 103 will identify a command to designate a region on the chest of the subject pointed by the hand. The area of the designated region 132 may be predefined or adjusted by the user depending on the attribute of the tissue to be determined within the designated region. For instance, if the detected object 130 is a hand, the designated region 130 may be a skin surface area that may contain a wound or a burn to be treated pointed to by the hand. Subsequently the attribute of the tissue may be an affected areas (cm²) of the skin. In another example, if a scalpel (object) is positioned over the heart, the designated region may span an area in which the surgeon will do an incision for a heart surgery. However, if the instrument is a cautery pen, the designated region may be limited to a narrower region to cauterize, for example, to stop bleeding from a blood vessel. Subsequently, the attribute may be determined as described before with reference to FIG. 1A and 1B.

The tissue of the subject within the designated region may be more precisely defined by an object indication provided by the detected external object. The object indication may define an open or nearly closed shape of the designated region drawn within the detecting field of view of the detector. FIG. 1C shows the external detected object 130 indicating a shape of the designated region 132. The detected object 130, in this case a hand has an endpoint, i.e., finger, that indicates a point of the shape of the designated region 132. The processor 103 may also be configured to recognize hand gestures for a more precise identification of the command and the designated region 132. If the hand moves within the detecting field of view of the detector 111 to continue indicating the shape of the designated region 132, the processor 103 detects successive points indicated by the object 130 as it moves; and connects the successive points to determine the shape of the designated region 132. The shape of the designated region 132maybe a closed or a nearly closed shape from which the attribute of superficial and/or deep tissues defined by of the designated region 132can be determined as described above with reference to FIG. 1A and 1B. In this example, of the designated region 132 indicated by the hand of the medical practitioner encompasses a boundary of a wound. Subsequently, the processor 103 may give more precise attributes of the wound, for example, its depth or severity.

Alternative the object 130 may not indicate a closed shape for the designated region but it may indicate an open shape to be followed to identify a new command to provide a recommendation to the medical practitioner. For example, when a surgeon will do an incision for a heart surgery, and the indicated shape is an open shape comprising a nearly vertical line, the processor may provide a recommendation on where to make the incision and the extension of it. In another example, if the instrument is a suturing needle, and the shape is a s- or z- shape, the processor may provide a recommendation of where on the skin to introduce the needle to close the suture. As mentioned before, the processor may use real-time or preoperative images/signals to determine the attribute of the tissues and also to provide recommendations. For example, the processor may use preoperative images to determine the location of the sternum with respect to the surface of the subject or the depth of the wound. In this manner, the processor may provide recommendations based on information that the medical practitioner may not easily see during surgery. It will be understood that the processor may be configured to provide recommendations either if the detected object pertains to the subject or not.

After determining the attribute of the tissue of the subject based within the designated region, the processor 103 will output an indication of the determined attribute. The indication may have any nature that the medical practitioner will recognize to understand the determined attribute. For instance, the output maybe auditory, such as voice message, or visual.

The apparatus 102 may comprise a screen or a display to provide the visual indication of the determined attribute. Alternatively, the apparatus comprises a projector configured to project on the surface of the subject 114 a projection image including the indication of the determined attribute of the tissue. Optionally, the projector may also project an indication of the designated region for the medial practitioner to better identify the tissue and designated region on the surface of the subject.

FIG. 2A schematically shows an apparatus 202 according to the present comprising a projector 106. The projector 106 disclosed herein is in general designed to project an image on the outside surface of the subject 114. Thus, the projector 106 can be located at a reasonable working distance from the subject 114, for example up to 3 meters. Projector 106 may generate projection light beams 112 from the detector field of view 115 (field of projection). It is noted that the projector 106 field of view may denote herein the area of the surface of the subject 114 onto which the projector 106 may project the projection image. It is further noted that the detector 104 field of view and the projector 106 field of view may overlap. The field of view of the projector 106 may also have a conical shape with a center (the apex) at the projector 106. While the detector 104 may detect objects anywhere in the volumetric field of view defined by the space between the detector and the surface of the subject, images projected by the projector 106 will only be visible until light beams 112 reach the surface of the subject. Thus, the projected image may be a 2D image projected on the surface within the field of view.

Because the projected image is projected from (exactly or approximately) the location of the detector 104, any deformations caused by the curvature of the surface may be avoided or strongly reduced, thereby reducing or making obsolete any image transformations to correct for such deformations. Alternatively, the projected 2D image may be corrected for the 3D surface on which it is projected. The apparatus 102 may further include a 3D sensor (not shown) such as a time-of-flight camera, stereoscopic camera, etc. to adapt the projected image to the 3D profile of the surface of the subject.

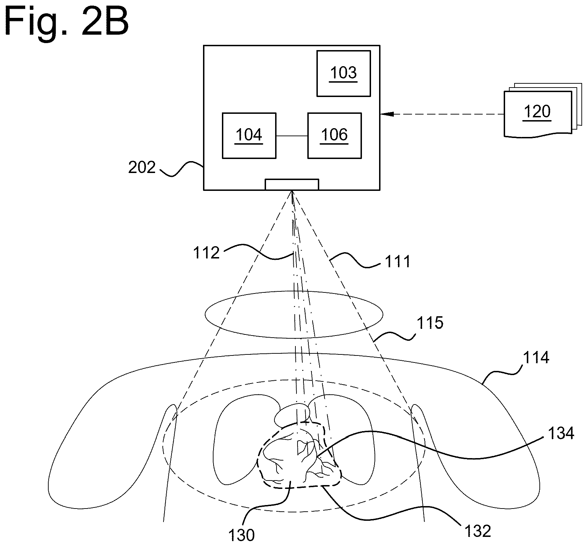

The processor 103 may be configured to generate a graphical representation of the object, the designated region, or the determined attribute and cause the projector to project the graphical representation onto the surface of the subject. As mentioned before, the detector 104 in FIG. 1B has detected at least part of a blood vessel as a detected object 130 and designated the region 132 of the heart. FIG. 2B schematically shows the apparatus of FIG. 2A detecting the same object as in FIG. 1B and additionally projecting an indication of the attribute of the tissue of the subject The attributes 134, i.e., location and length of the blood vessels may be indicated by the projector 106. The projected image (generated by light beams 112) representing the blood vessels may show a figure highlighting blood vessels or the heart. When the detected object is a burn, the projected image may project a borderline of the damaged tissue (attribute). If the detected object is an organ with abnormal tissue, the projected image may indicate what part of the organ contains the most abnormal tissue (attribute).

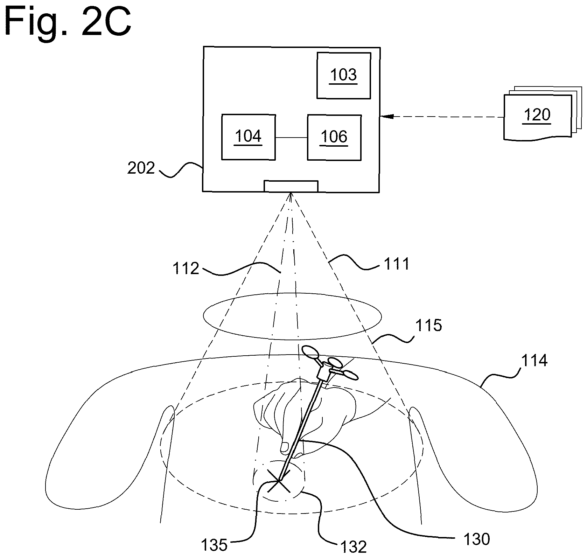

In some embodiments, the processor may offer recommendations based on the detected object and its attribute, without requiring the projection of an indication of the designated region. The processor identifies commands linked to the detected object and conducts attribute analysis to provide recommendations. For example, FIG. 2C schematically shows the apparatus of FIG. 2A assisting during a medical procedure. In this instance, the detected object 130 is a biopsy needle, and the designated region 132 corresponds to a skin area where the practitioner plans to insert the needle. The processor 103 determines the location attribute of a target tissue (e.g., liver) within the designated region 132. It then outputs a recommendation to the practitioner regarding the precise needle placement. This recommendation is conveyed through the projected image, which may display a cross or dot at the recommended insertion spot 135. The projector 106 aids the practitioner by projecting indications of the current and recommended depth and angle of the needle. This assistance is achieved through dynamically changing the shape's color and/or size based on factors like distance to the target tissue or needle angle.

The projected image may align with underlying structures of the subject. For example, blood vessels, an internal organ or cancerous tissue. When projecting the projected image on the surface of the subject, the graphical representation of the attribute or the designated region (showing an underlying structure) is projected on the surface of the subject. This allows medical practitioners to see information directly on the patient, without having to use separate display screens.

Optionally, the processor 103 may map real time measurements of the detector 104 to a pixel of the projected image. Detected signals may be transformed to pixels of the projected image. Similarly, the apparatus 102 may use the processor 103 to map pixels of the preoperative images 120 to pixels of the projected image. In this manner, each pixel of the projected image may be a visual representation of the preoperative or real-time information from the detector 104. For example, if the processor 103 has identified an organ (object) in an open cavity (designated region) by real-time measurements, the processor 103 may determine a size of the organ (attribute) by using preoperative MRI scan images. Subsequently the processor 103 may output an indication of the size of the organ by using the projector 106, for example a shape or a red color, which would assist the medical practitioner to identify inflammation. In the case of subcutaneous tissue, for example fat volume (attribute) in certain designated (subcutaneous) region, the indication of the volume of fat underneath the skin may be provided as a number in cm³ projected on the surface of the subject or may be provided as colored zones on the surface of the skin, wherein the intensity of the color may indicate thickness of a fat layer.

The projected image may be based on a plurality of images/signals detected by detector 104. For example, if the apparatus 102 includes a thermal image detector and an infrared image detector, e.g., filtered for Indocyanine Green (ICG), the processor 103 may identify a section of the skin surface (object) at the abdominal region (designated region), determine a skin surface temperature (attribute) of the subject 114 and output an indication of the temperature by causing the projector 106 to project an image that represents and indicates a presence of the dye in subject 114.

Although the detector 104 may be configured to detect radiation with any suitable wavelength range, the projector may be configured to emit light in the visible light spectra, so that the image that is projected on the subject surface is visible to the human eye. The projector 106 may comprise of a laser projector or a plurality of light generating elements, such as LEDs, arranged in a matrix on a substrate. For example, projector 106 may be a laser projector configured to project the projected image using coherent parallel beams. As another example, if projector 106 may be an LED projector, where each light generating element may correspond to a pixel of the output image.

Processor 103 may be configured to execute the mapping algorithm so that the projected image displays the information on the subject surface at the same position where the designated region 132 is. The mapping algorithm may allow for a margin of error and the margin of error may depend on the type of detector. In other words, the projected image may not be in exactly the same position on subject surface as the detected image/signals.

Alternatively, in another example, the projected image may include more than a graphical representation of the detected image/signals. The projected image may also include data related to the attribute of the tissue and project the data on the surface of the subject. The projected image may also include a name and/or a color overlay of the identified object when the object pertains to the subject, e.g., the object is a blood vessel or nerve, etc. For instance, if the detector can detect movement of red blood cells in the blood vessel (object), the projected image may incorporate, in the graphical representation, an overlay of a flow (attribute) over the blood vessel, optionally multiple blood vessels with different flows of blood cells may be represented in the projected image by different colors. This provides medical practitioners and users a more complete understanding by combining visual indications with attribute-related insights, further assisting in a real-time decision-making process. In another example when the detector is a camera, and the procedure is a surgical procedure, such as breast reconstruction, the projected image comprises guidelines to assist the medical practitioner to improve on symmetry in the reconstruction of the breast (designated region). In an even further example, during an internal fixation of a bone fracture, the detected object may be at least part of a bone to be repaired, the designated region may be the complete bone, the attribute may be the location and/or orientation of the bone, and the projected image may comprise recommended placement for rods and screws. The projected image may further include an indication of the relative position of the bone from a surgical tool, e.g. project an angle between a saw and the bone to be used during e.g. osteotomy.

The processor may also use Artificial intelligence-based algorithms to cause the projector to create the projected image as described above.

The apparatus may comprise a memory to store the determined attribute(s) for future reference or analysis. The memory may act as a repository for the attribute(s) that are determined during the course of a procedure. This memory facilitates the storage of attribute-related information for subsequent reference or in-depth analysis. For example, the processor may be able to determine of the tissues off-line after a surgery.

The memory may hold instructions for the processor to follow. The detector may simultaneously detect multiple objects, supplemented by the memory's repository of instructions derived from machine learning and AI algorithms. These algorithms enable the processor to recognize objects and identify corresponding commands for each one.

In some embodiments, the processor maybe associated to a database holding information collected from prior procedures facilitated by the same apparatus. The database may contain historical attribute data and related details. The processor may access the database to improve the attribute's accuracy and to determine attributes for each detected object more effectively. This enhances the apparatus's capabilities, providing medical practitioners and users with information during complex medical procedures.

Some or all aspects of the invention may be suitable for being implemented in form of software, in particular a computer program product. This applies for example to the software of the control unit 204 and the software to control the image detector and projector, and the software to process the detected image and convert it into the projected image. The computer program product may comprise a computer program stored on a non-transitory computer-readable media. Also, the computer program may be represented by a signal, such as an optic signal or an electro-magnetic signal, carried by a transmission medium such as an optic fiber cable or the air. The computer program may partly or entirely have the form of source code, object code, or pseudo code, suitable for being executed by a computer system. For example, the code may be executable by one or more processors.

For example, certain embodiments of the invention may be implemented as a computer-implemented method. Such a method may comprise detecting, by a detector having a detecting field, at least part of a subject and an object in the detecting field; identifying, by a processor system, a command based on the detected object, wherein the command designates a region on the surface of the subject; determining, by the processor system, an attribute of a tissue of the subject within the designated region; and outputting, under control of the processor system, an indication of the determined attribute. The method may further comprise projecting, by a projector, on the surface of the subject an indication of the designated region and/or the indication of the determined attribute of the tissue. For example, the object is external to the subject, and the object indicates a point of a shape of the designated region. The method may comprise detecting successive points, on the surface of the subject, indicated by the object as the object moves with respect to the subject; and connecting the successive points to determine (an outline of) the shape of the designated region. The method may comprise projecting, by the projector, an indication of the shape of the designated region insofar the points on the surface of the subject have been detected. The shape of the designated region may be a closed shape or a nearly closed shape. Determining the attribute of the tissue may comprise determining the attribute of superficial and/or deep tissue of the subject on and/or below an area defined by the designated region. The step of detecting the object may comprise detecting at least part of a hand or an instrument as the object.

Alternatively, the object may be a part of a body of the subject. The method may comprise determining a shape of the object within the designated region.

In order to determine the attribute, the method may comprise performing real-time measurements of the designated region and/or mapping the designated region on preoperative images containing data representing the subject.

The method may further comprise generating a graphical representation of the determined attribute and cause the projector to project the graphical representation onto the surface of the subject. Herein, the projected graphical representation of the determined attribute may be aligned with the designated region.

Features described in respect of the apparatus may be advantageously applied to the method, and vice versa.

The examples and embodiments described herein serve to illustrate rather than limit the invention. The person skilled in the art will be able to design alternative embodiments without departing from the spirit and scope of the present disclosure, as defined by the appended claims and their equivalents. Reference signs placed in parentheses in the claims shall not be interpreted to limit the scope of the claims. Items described as separate entities in the claims or the description may be implemented as a single hardware or software item combining the features of the items described.

Modifications and alternative implementations of some parts or elements are therefore possible, and are included in the scope of protection as defined in the appended claims.

Certain aspects are defined in the following clauses.
1. An apparatus comprising:
   a detector having a detecting field and configured to detect at least part of a subject in the detecting field and detect an object in the detecting field; and
   a processor configured to:
      identify a command based on the detected object, wherein the command designates a region on the surface of the subject;
      determine an attribute of a tissue of the subject within the designated region; and
      output an indication of the determined attribute.
2. The apparatus of clause 1, further comprising a projector configured to project on the surface of the subject an indication of the designated region and/or the indication of the determined attribute of the tissue.
3. The apparatus of clause 1 or 2, wherein the object is external to the subject, and wherein the object indicates a point of a shape of the designated region, and the processor is configured to:
   detect successive points, on the surface of the subject, indicated by the object as the object moves with respect to the subject; and
   connect the successive points to determine the shape of the designated region.
4. The apparatus of clause 3, wherein the projector is configured to project an indication of the shape of the designated region insofar the points on the surface of the subject have been detected.
5. The apparatus of clause 3 or clause 4, wherein the shape of the designated region is a closed shape or a nearly closed shape, and wherein determining the attribute of the tissue comprises determining the attribute of superficial and/or deep tissue of the subject on and/or below an area defined by the designated region.
6. The apparatus of any preceding clause, wherein the detector is configured to detect at least part of a hand or an instrument as the object.
7. The apparatus of clause 1 or clause 2, wherein the object is a part of a body of the subject, and the processor is further configured to determine a shape of the object within the designated region.
8. The apparatus of any preceding clause, wherein the attribute of the tissue comprises one or more of: name, temperature, size, area, volume, location, and type of tissue.
9. The apparatus of any preceding clause, wherein the processor is configured to determine the attribute of visceral, epithelial, connective, adipose, muscle, nervous, or abnormal tissue of the subject.
10. The apparatus of any preceding clause, wherein the attribute of the tissue is determined based on real-time measurements of the designated region and/or based on mapping the designated region on preoperative images containing data representing the subject.
11. The apparatus of clause 10, wherein the preoperative images comprise images from one or more medical imaging techniques.
12. The apparatus of any preceding clause, wherein the attribute of the tissue is determined by a recognition algorithm comprising Artificial Intelligence.
13. The apparatus of any preceding clause, wherein the attribute of the tissue is determined momentarily and/or over time.
14. The apparatus of any preceding clause, wherein the detector comprises an image sensor and/or a position sensor to detect a position of the object.
15. The apparatus of any preceding clause, wherein the apparatus further comprises a memory to store the determined attribute of the tissue for future reference or analysis.
16. The apparatus of any preceding clause, wherein the detector is able to detect multiple objects simultaneously, and the processor is configured to identify commands and determine attributes for each detected object individually.
17. The apparatus of any preceding clause, wherein the processor is further configured to analyze the attribute of the tissue and provide a recommendation for a procedure based on the analysis.
18. The apparatus of any preceding clause, wherein the processor is configured to generate a graphical representation of the determined attribute and cause the projector to project the graphical representation onto the surface of the subject.

## Claims

1. An apparatus comprising:
a detector having a detecting field and configured to detect at least part of a subject in the detecting field and detect an object in the detecting field; and
a processor configured to:
identify a command based on the detected object, wherein the command designates a region on the surface of the subject;
determine an attribute of a tissue of the subject within the designated region; and
output an indication of the determined attribute.

2. The apparatus of claim 1, further comprising a projector configured to project on the surface of the subject an indication of the designated region and/or the indication of the determined attribute of the tissue.

3. The apparatus of claim 1 or 2, wherein the object is external to the subject, and wherein the object indicates a point of a shape of the designated region, and the processor is configured to:
detect successive points, on the surface of the subject, indicated by the object as the object moves with respect to the subject; and
connect the successive points to determine the shape of the designated region.

4. The apparatus of claim 3, wherein the projector is configured to project an indication of the shape of the designated region insofar the points on the surface of the subject have been detected.

5. The apparatus of claim 3 or claim 4, wherein the shape of the designated region is a closed shape or a nearly closed shape, and wherein determining the attribute of the tissue comprises determining the attribute of superficial and/or deep tissue of the subject on and/or below an area defined by the designated region.

6. The apparatus of any preceding claim, wherein the detector is configured to detect at least part of a hand or an instrument as the object.

7. The apparatus of claim 1 or claim 2, wherein the object is a part of a body of the subject, and the processor is further configured to determine a shape of the object within the designated region.

8. The apparatus of any preceding claim, wherein the attribute of the tissue comprises one or more of: name, temperature, size, area, volume, location, and type of tissue.

9. The apparatus of any preceding claim, wherein the processor is configured to determine the attribute of visceral, epithelial, connective, adipose, muscle, nervous, or abnormal tissue of the subject.

10. The apparatus of any preceding claim, wherein the attribute of the tissue is determined based on real-time measurements of the designated region and/or based on mapping the designated region on preoperative images containing data representing the subject.

11. The apparatus of any preceding claim, wherein the detector comprises an image sensor and/or a position sensor to detect a position of the object.

12. The apparatus of any preceding claim, wherein the apparatus further comprises a memory to store the determined attribute of the tissue for future reference or analysis, or wherein the attribute of the tissue is determined by a recognition algorithm comprising Artificial Intelligence.

13. The apparatus of any preceding claim, wherein the detector is able to detect multiple objects simultaneously, and the processor is configured to identify commands and determine attributes for each detected object individually.

14. The apparatus of any preceding claim, wherein the processor is further configured to analyze the attribute of the tissue and provide a recommendation for a procedure based on the analysis.

15. The apparatus of any preceding claim, wherein the processor is configured to generate a graphical representation of the determined attribute and cause the projector to project the graphical representation onto the surface of the subject.
